# EUROPEAN PATENT APPLICATION

(11) **EP 1 216 675 A1**
(43) Date of publication of application: **26.06.2002**
(21) Application number: 00127795.3
(22) Date of filing: 19.12.2000
(51) Int. Cl.: A61F 13/42

(54) **Indicator means for detecting faecal matter**

(71) Applicant: SCA Hygiene Products AB, 405 03 Göteborg (SE)
(72) Inventor: Kling, Robert, 51163 Skene (SE)
(74) Representative: Harrison, Michael Charles

(57) **Abstract**

An indicator (11) particularly for use in disposable diapers is provided for detecting the presence of faecal matter. The indicator has at least one indicator portion (12-14; 22; 31, 32) which is triggered into producing a signal upon detection of a gas given off by the faecal matter.

## Description

### Field of the invention:

The present invention relates to an indicator means for detecting the presence of faecal matter. In particular, such a detector may be used with an absorbent product for the absorption of bodily exudates, such as diapers or other absorbent garments of a disposable or re-usable type.

### Background to the invention:

Indicator means for indicating the presence of faeces in absorbent products are known. For examples WO-A-99/23985 discloses fibre optic strands having one end within an absorbent core and an opposite end adjacent the back sheet of the product. In this way, an attendant can view whether the product is soiled or clean without inspecting the absorbent product internally.

Similarly, US-A-5 078 708 discloses a diaper having distinct transparent areas in the back sheet allowing faecal matter to be viewed from an external location.

WO-A-96/20681 discloses an indicator means whereby the presence of faeces is detected dependent upon whether certain electrical circuits in an absorbent product liner have been short-circuited.

The aforementioned types of indicator means rely on the faecal matter being in a particular location in an absorbent product, either to allow it to be physically visible or so as to be able to short-circuit a particular part of a circuit. Faecal matter may however avoid such locations, especially when the absorbent product becomes wrinkled or otherwise deformed since this forms channels or folds in the absorbent product core or sheet structure which direct the faecal material along a different path. Similarly, the wrinkles or folds in the product may prevent the faecal material from reaching certain areas at all. Faecal matter may not therefore always lie exactly where it is visible or readily detectable. The presence of undetected faecal matter can however lead to the drawbacks of discomfort and/or skin damage for example.

The main object of the present invention is to provide a solution to the aforementioned problems.

It is a secondary object of the invention that the means should be cheap to both manufacture and to include in an absorbent product, such that the required means can be unitary with a disposable absorbent product.

### Summary of the invention

The main objective is achieved by an indicator means having the features defined in claim 1. Preferred features of the invention are defined in the dependent claims.

Further features of the invention will be apparent to the reader of this specification.

In accordance with the invention, an indicator means is provided which reacts to the presence of one or more gases given off by faecal matter.

It will be understood that the concept of the invention can be used for indicator means attached to disposable or reusable absorbent products. The invention may however also be applied for use in beds, whereby the indicator means could be arranged, for example, on an article of bed clothing such as a sheet or blanket. The latter may be useful particularly if the indicator is to provide some type of alarm warning. The invention may also be applied to seats of various types (car seats, child car seats, chairs of various types etc.,) by being attached to a seat or seat cover for same. More generally it will be appreciated that the invention may find application in any form of support surface or cover of said support surface on which an incontinent wearer may sit or lie.

The indicator means of the present invention functions by being triggered by a gaseous product of faecal material. For example, mercaptans or amines are both suitable gases for detection. However it is has been found currently more costly to provide an indicator means which establishes the presence of mercaptans, even though mercaptans may be easily perceived by the human nose due to their often distinctive sulphurous odor. Amine gases on the other hand are less readily detectable by humans, but have been found to be particularly suitable as a trigger means in the present application.

Although gas detectors in many forms are known and have been used in other unrelated applications for many years, the idea of detecting amines or mercaptans for example so as to provide an indirect indication of the presence of faecal matter involves a unique concept and application. In the present case, the volatile nature of the gases given off by faecal matter allow the indicator means to be placed at a location in an absorbent product, for example, which will be in an area reached by the gases. In an absorbent product, this would appropriately be in one or both of the end 1/3 portions of the product as seen in a lengthwise direction, implying a placement of the indicator means in the front or rear waist regions. Alternatively or additionally to indicator means in the waist region(s), indicator means in the area around the crotch (e.g. proximate the leg cutouts) however may also be used for example.

Amine indicator means suitable for the present invention by virtue of their low cost may for example include amine detectors of the paper strip type, which comprise a paper substrate impregnated or coated with an indicator substance that changes colour upon contact with amines. Typically amines such as ammonia may be present, not only as result of faeces alone but also due to the presence of faeces and urine together. Such paper strips might be similar to the Chemcassette® tape type which is available from Zellweger Analytics Inc., IL 60069, USA. These gas detector tapes can be made more or less specific to the gas to be detected.

Further gases which give a good indication of the presence of faecal matter are mercaptans and volatile fatty acids for example.

Mercaptan indicator means may be in the form of a cumulative concentration sensor, whereby indication of the presence of mercaptans requires a cumulative build up of mercaptans over a period of time before a cumulative threshold is exceeded. Such systems are currently relatively expensive and thus more likely to be used only in a re-usable environment at the present time. Thus, in terms of absorbent products, mercaptan indicators might typically be arranged as a re-usable item which could be removed from the absorbent article when soiled and then attached to a further article.

Mercaptans sensors are available for example from the company Otis Instruments. One such detector is the Otis Model OI-240 which is a solid-state hydrogen sulfide detection element having a length of approximately 8 mm (0.316 inches) and a width of 6.9 mm (0.271 inches) and a thickness of only 2 mm (0.080 inches). This detector is very mercaptan-specific and can provide a detection of mercaptans of between 1 and 3 ppm.

Such mercaptan detectors require a voltage source of about 7.0V and a current of some 165mA, which means that a suitable power source should be provided. However power sources are these days very light and very small and the cost of same is decreasing rapidly such that total additional cost of a detector and power source to an absorbent article can be kept low. Similarly, the size of such units are very small so that they can be made inconspicuous if required. When these detectors are sold as a re-usable unit for attachment to absorbent articles of differing types including both disposable and non-disposable absorbent articles, the cost factor is less important of course.

The indicator means itself includes at least a part thereof which functions as an indicator portion to provide a signal to the wearer or an attendant that faeces is present. Thus the indicator means may include a combined indicator/detector portion (i.e. detection and indication at the same location) or a detector portion and an indicator portion which are separated positionally, whereby the detector portion might be a gas sensor connected by some type of transport means to an indicator portion in the form of a visible or audible signal means placed for example on the outside of an absorbent product.

In order to prevent false detections occurring by very small quantities of gas being present (e.g. due to the temporary presence of flatus and not faeces), the indicator means may be provided with a gate means which is set only to allow activation of the indicator portion of the indicator means when a predetermined threshold is reached. The gate means itself is normally in a first, passive or rest state but changes upon the predetermined threshold being exceeded.

The threshold chosen depends on the parameter being evaluated for deciding when an indication to an observer should be provided. The threshold parameter used could for example be contact time of the gas with a gas sensor or could be combined with gas concentration.

In this way, an indication can be provided based on a predetermined set of conditions, which allows an absorbent product designer the possibility of varying the predetermined gate parameters so as to suit different groups of user and size of product.

It will be appreciated that the present invention will find its main use in absorbent products where faeces is most commonly present. Such absorbent products include especially diapers (for adult, infant and baby use), absorbent pants having an absorbent core (e.g. so-called training pants) as well as diaper chassis members and other incontinence products which have replaceable absorbent inserts.

### Brief description of the drawings

The invention will now be explained in more detail with reference to certain non-limiting embodiments thereof and with the aid of the accompanying drawings, in which:
- Fig. 1: shows a diaper in a flattened state seen from above and viewed from the body liner (top sheet) side,
- Fig. 2: shows the upper (front) waist part of the diaper illustrated in Fig. 1, whereby the indicator means is in the form of a coating,
- Fig. 3: shows a view similar to Fig. 2, in which there are two indicator portions, both positioned outside the contour of the diaper and connected to a detector portion within said contour,
- Fig. 4: shows a view similar to Fig. 2, in which the indicator means is positioned between the top sheet and the back sheet, and
- Fig. 5: shows a cross-sectional view taken along line V-V in Fig. 4.

### Detailed description of preferred embodiments:

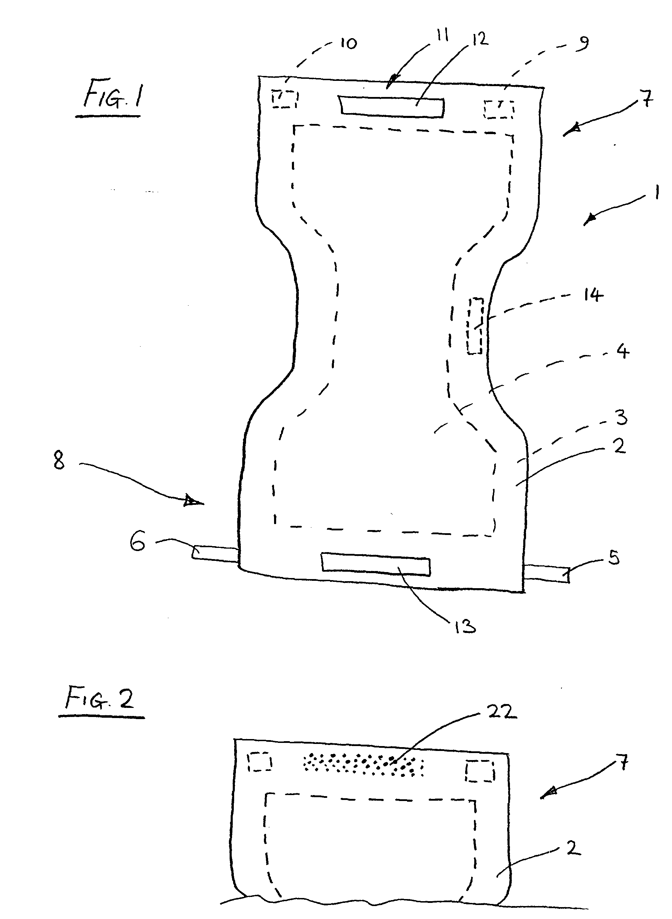

In Fig. 1, the absorbent product 1 in the form of a diaper has been illustrated in a flattened form and is depicted from the inner side thereof.

Reference numeral 2 denotes a liquid and gas permeable top sheet, which is connected to a back sheet 3 of liquid-impermeable material typically consisting of a semi-transparent PE film. Such top sheet and back sheet materials are common in the field of current day diapers. The back sheet in particular (which face outwards towards an observer when the diaper is worn) is semi-transparent and allows the underlying elements to be visibly discerned to a certain degree. As will be understood from the following, the visibility of an underlying indicator portion may be further improved if the transparency of the back sheet is enhanced e.g. by providing transparent windows in same.

An absorbent core 4 of, for example, cellulosic material such as wood pulp fibres or the like, typically in an hour glass form, is positioned between the top sheet 2 and the back sheet 3. The absorbent core may be enveloped by a thin pervious layer such as a non-woven sheet (not shown) in some cases. The top sheet 2 and back sheet 3 are then sealed around their contacting peripheries, preferably by means of welding or adhesive, to thereby form an envelope enclosing said core 4 and, if present, its enveloping pervious layer. The core 4 thus normally has dimensions which are smaller in both the length and width directions of the article, as shown.

Attachment means, such as hook and loop type mechanical fastening means or tape means 5 and 6, are provided at respective front and rear waist ends 7 and 8 of the article, whereby end 7 is intended to be the front waist portion of the product 1 and end 8 the rear waist portion, when applied to the wearer. In such an arrangement the tabs 5 and 6 could for example form adhesive tabs adapted to attach to front waist part of the back sheet at locations 9 and 10, or hook tabs which are placed on the outer side of the back sheet 3, whilst tabs 9 and 10 could be loop tabs designed to engage with respective tabs 5 and 6 after the product has been passed through the legs of a wearer and up to the waist for attachment around the waist as is normal in the art.

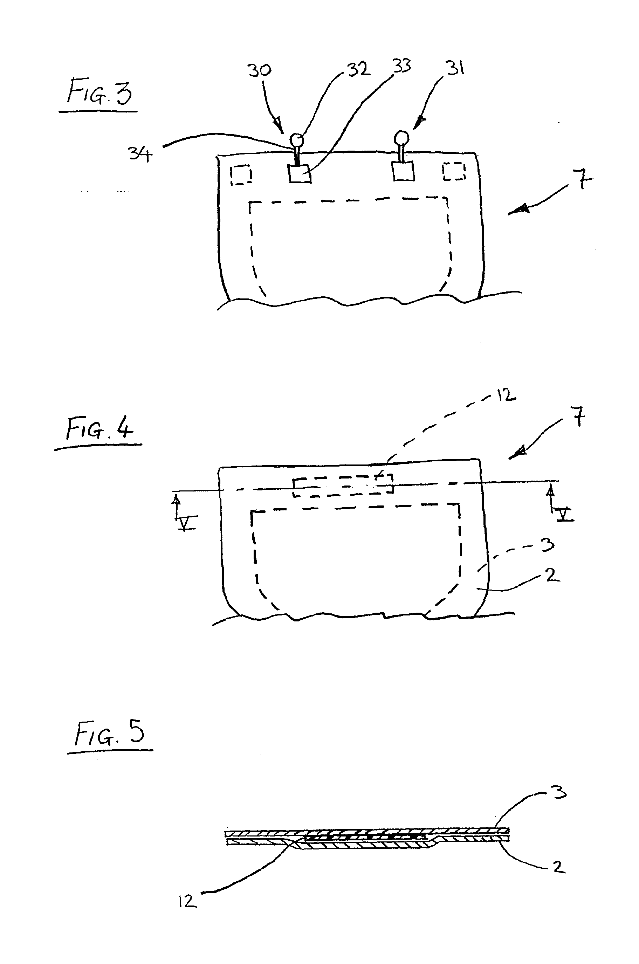

An indicator means denoted generally as 11 is attached to e.g. the inner side of top sheet 2 of the diaper. Attachment of same may however be on the hidden side of the top sheet 2, between the top sheet and back sheet (see e.g. Fig. 4).

The indicator means 11 may be placed at one or both ends 7, 8 of the absorbent article 1 and may additionally or alternatively be positioned in one or both leg cut-out portions proximate the crotch region. Indicator means in the form of strips 12, 13 and 14 depict examples of the placement of such strips, whereby the strip 14 is placed between the top and back sheet 2 and 3 for reasons of comfort.

In the embodiment shown, the strips 12, 13 and 14 may be strips of substrate such as paper which are impregnated or coated with a chemical substance able to react with, for example, amine gases. As a result of such reaction the chemical may change colour to thereby provide a distinct visual indication that amine gases are present. The change of colour can be viewed through the transparent or semi-transparent back sheet 3. Similarly however, the strips 12 and/or 13 may be viewed by partial opening of the waist portions of the diaper if the back sheet is not sufficiently translucent, but without needing to remove the diaper or resort to a more thorough inspection. Particularly in the case of incontinent adults, such an arrangement requiring opening of the waist portion may be beneficial since it can assist in preserving the privacy of the wearer by not providing an external warning to all observers.

As will be understood from the aforegoing, the exact position of the indicator means 11 may be varied to a great degree, without preventing gas from faeces being reliably detected and indicated, due to the relative volatility of these gases.

Fig. 2 shows an alternative embodiment depicting only the front waist portion 7 of the diaper of Fig. 1, in which the strip 12 has been replaced by a gas-indicating coating 22 applied directly to the top sheet 2. The coating may be suitably applied in the form of a spray so as to take account of the flexible nature of the top sheet and to thereby improve comfort during wearing. Such sprays are known per se and will change colour upon detection of amines for example. Similarly the coating may alternatively be applied to the back sheet 3 since the top sheet 2 is permeable and thus will allow gas from faecal matter to pass through the top sheet to reach the back sheet.

A third embodiment is shown in Fig. 3. In this case, the indictor means comprises two parts, a first indicator means 30 and a second indicator means 31, each of which is substantially identical. Means 31 comprises an indicator portion 32 for providing a visible or audible indication, located at a distance from a detection portion 34 which actually detects (i.e. senses) the presence of gas. Parts 32 and 33 and joined together by means of a joining means 34 for transmitting a signal produced by detector portion 33 to said indicator portion 32.

As an example, detector portion 33 may be constituted by a mercaptan sensor, which are per se known in the art, which upon detecting a certain level of mercaptans over a period of time, will supply a signal to the indicator means (e.g. an electrical signal) to actuate the indicator portion 32. Joining means 34 may thus simply constitute a flexible electrical conductor of some type. As will be clear, the joining means may be made as long as required so that the indication means (e.g. an electrically-powered display) can be positioned outside a wearer's clothing.

Where the detector portion is designed to detect mercaptans for example, it may be suitable if a preset threshold level is made in the detection portion such that no signal is sent to the indicator portion until an accumulated level of mercaptans has been achieved. Such could for example correspond to a predetermined ppm count (e.g. 5 to 10 ppm) which is thus a cumulative threshold level.

The indicator means 30, 31 can be arranged to be removably attached to the absorbent article or other surface to which it is applied. For example, hook attachment strips of a hook and loop type fastening means could be attached to the rear of the detector portions with the hooks pointing outwardly such that they can be secured removably to the top sheet by contact therewith. Preferably however the indicator means should be made disposable along with the diaper itself.

Fig. 4 and the cross-sectional view of same in Fig. 5 show a further embodiment which differs from the embodiment shown in Fig. 1 only by means of the strip 12 having been placed between the top sheet 2 and the back sheet 3. The strip 12 may be either fixed to the top sheet or the back sheet, but has been shown fixed to the back sheet 3 in Fig. 5.

Further embodiments will be readily understood by the skilled person upon reading the aforegoing and are intended to be encompassed within the scope of the invention as defined by the appended claims

## Claims

1. An indicator means (11) for detecting the presence of faecal matter, wherein said indicator means comprises at least one indicator portion (12-14; 22; 31, 32), **characterized in that** said indicator portion provides a signal upon detection of a gas given off by said faecal matter.

2. An indicator means according to claim 1, **characterized in that** said indicator means (11) is triggered by the presence of mercaptans and/or amines and/or volatile fatty acids.

3. An indicator means according to claim 2, triggered by the presence of amines, **characterized in that** said indicator means (11) includes a chemical which changes colour as a result of contact with amine gas.

4. An indicator means according to claim 3, **characterized in that** said chemical is applied in the form of a coating.

5. An indicator means according to claim 4, **characterized in that** said chemical is applied in the form of a spray coating (22) to any one of a back sheet (3), top sheet (2) and absorbent core (4) of an absorbent product (1).

6. An indicator means according to any one of the preceding claims, **characterized in that** said indicator portion (12-14; 22; 31, 32), provides a signal after a predetermined threshold has been exceeded.

7. An indicator means according to claim 6, **characterized in that** said predetermined threshold is a cumulative threshold whereby the total amount of said gas detected by a detector portion (33) of said indicator means over a period of time is used to determine whether the predetermined threshold has been exceeded.

8. An indicator means according to any one of claims 1 to 7, **characterized in that** said indicator means (11) is attached in an absorbent product of the type used for absorbing bodily exudate.

9. An indicator means according to claim 8, **characterized in that** said absorbent product is a diaper (1) or a pair of absorbent pants.

10. An indicator means according to any one of claims 1 to 7, **characterized in that** said gas detector is attached to a support surface or an article covering said support surface.

11. An indicator means according to claim 10, **characterized in that** said support surface is a bed or an article of bed clothing.

12. An indicator means according to claim 10, **characterized in that** said support surface is a seat or a seat cover.
